# EUROPEAN PATENT APPLICATION

(11) **EP 4 356 906 A1**
(43) Date of publication of application: **24.04.2024**
(21) Application number: 23204633.4
(22) Date of filing: 19.10.2023
(51) Int. Cl.: A61K 31/185, A61K 9/48, A61K 31/352, A61K 31/353, A61K 31/525, A61K 31/7068, A61P 27/06

(54) **COMPOSIZIONE NUTRACEUTICA AD EFFETTO NEUROPROTETTIVO PER IL TRATTAMENTO DEL GLAUCOMA**

(30) Priority: 20.10.2022 IT 202200021624
(71) Applicant: SIFI S.p.A., 95025 Aci Sant'Antonio (CT) (IT)
(72) Inventor: LA ROSA, Luca Rosario, 95045 Misterbianco (IT); PEPE, Veronica, 95030 Mascalucia (IT); ZAPPULLA, Cristina Maria Concetta, 95127 Catania (IT)
(74) Representative: Cattaneo, Elisabetta

(57) **Abstract**

The present invention concerns a nutraceutical composition with a neuroprotective effect for the treatment of glaucoma, comprising riboflavin, epigallocatechin gallate, citicoline, forskolin, and homotaurine.

## Description

### FIELD OF THE INVENTION

The present invention concerns a nutraceutical composition for the treatment of glaucoma.

### STATE OF THE ART

Glaucoma is a multifactorial neurodegenerative disease characterized by optic neuropathy, visual field loss, and retinal degeneration with apoptosis of retinal ganglion cells (RGCs), a phenomenon that leads to vision loss. To date, glaucoma is a leading cause of irreversible blindness and the World Health Organization forecasts that it will affect more than 112 million people by 2040.

The mechanisms through which glaucoma develops are still partly unknown, although numerous risk factors associated with the disease have been identified.

Among these, the best known is certainly the increase in intraocular pressure (IOP, IntraOcular Pressure). It has in fact been known for some time that in the presence of high IOP, retinal ganglion cells are subject to degenerative processes and can even undergo cell death, thus leading to the consequent progressive vision loss in the affected subject.

However, it is also known that RGCs can be damaged and undergo cell death also through mechanisms other than the increase in IOP and only partially known; for example, through excessive production of oxygen free radicals (hydrogen peroxide) followed by oxidative stress and glutamate-induced neurotoxicity.

Indeed, several studies have shown that free radicals can cause RGCs death through inhibition of key enzymes of the tricarboxylic acid cycle, mitochondrial electron transport chain, and calcium homeostasis, leading to defective energy metabolism. Furthermore, increased levels of oxidative stress markers have been observed in the aqueous humor of patients with primary open-angle glaucoma (POAG) and primary closed-angle glaucoma (PACG).

### SUMMARY OF THE INVENTION

Although research has made in recent years enormous progress in the identification of drugs that can be used for this purpose, the Applicant has perceived that there is a pressing need to identify and develop new and more effective therapeutic strategies in the treatment of glaucoma, both for its greater incidence on the population, and due to the multiplicity of causes and risk factors, which may require the adoption of new therapeutic approaches, both as independent drug and as an adjuvant to be added to those already existing which aim to protect RGCs, for example by reducing IOP or counteracting the effect of free radicals.

The object of the present invention is therefore to provide a nutraceutical composition, capable of offering a new and more effective therapeutic possibility for the treatment of glaucoma.

To this end, the Applicant has surprisingly found that the combined use of riboflavin with other compounds already known in the treatment of glaucoma, such as epigallocatechin gallate, citicoline, forskolin, and homotaurine, is able to offer an unexpectedly powerful tool thanks to the unexpected and surprising capacity of riboflavin to interact synergistically with said compounds and exert a greater neuroprotective effect therewith.

In its first aspect, therefore, the present invention refers to a nutraceutical composition comprising riboflavin, epigallocatechin gallate, citicoline, forskolin, and homotaurine.

Said composition has in fact proven to be capable of exerting a surprisingly high neuroprotective effect, thus offering a new and effective therapeutic treatment of glaucoma.

In a further aspect, therefore, the present invention relates to the composition according to the first aspect of the invention, for use in the treatment or prevention of glaucoma.

Thanks to the unexpected and surprising ability of riboflavin to interact synergistically with other compounds already known in the treatment of glaucoma, such as epigallocatechin gallate, citicoline, forskolin, and homotaurine, and exert a greater neuroprotective effect therewith, the combined use of riboflavin with said compounds offers an unexpectedly powerful tool in preventing the degeneration of retinal ganglion cells.

Therefore, in a further aspect, the present invention also refers to the use of riboflavin in the treatment of glaucoma, as an enhancing agent of the protective effect on retinal ganglion cells of a compound selected from the group consisting of epigallocatechin gallate, citicoline, forskolin, homotaurine, and binary, ternary, and quaternary mixtures thereof.

The advantages and characteristics of said further aspects have already been highlighted with reference to the first aspect of the invention and are not repeated here.

### BRIEF DESCRIPTION OF THE FIGURES

Figure 1 shows the results of the cell viability assay according to Example 1;
Figure 2 shows the neuroprotective effect results evaluated in terms of cell viability according to Example 2; and
Figure 3 shows the neuroprotective effect results evaluated in terms of brain-derived neurotrophic factor (BDNF) expression according to Example 3.
Figure 4 shows the neuroprotective effect result of vitamin B2 and the formulations COEF, COEF + B2, CONF and P-INV of Example 4. Data are expressed as mean ± s.e.m. ***p≤0.001 and ****p≤0.0001 vs. CTRL; °p≤0.05, °°p≤0.01, °°°p≤0.001 and °°°°p≤0.0001 vs. P-INV; ▪p≤0.05 vs. COEF + B2. One-way ANOVA followed by Dunnett's *post-hoc* test.
Figure 5 shows the neuroprotective effect results of homotaurine, forskolin, vitamin B2 and the COEF + B2 formulation of Example 4. Data are expressed as mean ± s.e.m. ****p≤0.0001 vs. CTRL; ••••p≤0.0001 vs. COEF + B2. One-way ANOVA followed by Dunnett's *post-hoc* test.

### DETAILED DESCRIPTION OF THE INVENTION

In its first aspect, the present invention relates to a nutraceutical composition comprising riboflavin, epigallocatechin gallate, citicoline, forskolin, and homotaurine.

Although, in recent years, research has made enormous progress in the identification of drugs that can be used in the treatment of glaucoma, the Applicant has in fact perceived the pressing need to identify and develop new and more effective therapeutic strategies for this purpose, both due to the ever-increasing incidence of this pathology in the population, and for the multiplicity of causes and risk factors connected with the onset of glaucoma, as well as for the benefits related to the adoption of new therapeutic approaches, both as an independent drug and as an adjuvant to be added to the already existing ones aiming at protecting RGCs, for example by reducing IOP or counteracting the effect of free radicals.

For this purpose, the Applicant has surprisingly found that the combined use of riboflavin with other compounds already known in the treatment of glaucoma, such as epigallocatechin gallate, citicoline, forskolin, and homotaurine, is able to offer an unexpectedly powerful tool thanks to the unexpected and surprising capacity of riboflavin to interact synergistically with said compounds and exert a greater neuroprotective effect therewith, thus being able to offer a new and more effective therapeutic treatment of glaucoma.

Within the scope of the present description and in the subsequent claims, all numerical quantities indicating amounts, parameters, percentages, and so on, are to be understood as preceded in all circumstances by the term "about" unless otherwise indicated. Furthermore, all ranges of numerical quantities include all possible combinations of the maximum and minimum numerical values and all possible intermediate ranges, in addition to those specifically indicated below. The present invention may exhibit in one or more of its aspects one or more of the preferred characteristics reported below, which can be combined with each other according to application needs.

Preferably, the composition according to the present invention comprises from 1.21 to 0.81% (w/w), more preferably from 1.11 to 0.91% (w/w), optimally 1.01% (w/w) of riboflavin (vitamin B2).

Preferably, the composition according to the present invention comprises from 8 to 5.33% (w/w), more preferably from 7.33 to 6.0% (w/w), optimally about 6.67% (w/w) of epigallocatechin gallate.

Epigallocatechin gallate is a natural polyphenol, present for example in tea leaves. In one embodiment of the invention, for the preparation of the composition according to the present invention, epigallocatechin gallate is advantageously used in the form of green tea leaf extract (*Camellia sinensis L*.). In a particularly preferred embodiment, epigallocatechin gallate is in the form of 95% ECG green tea leaf dry extract.

Preferably, the composition according to the present invention comprises from 15.51 to 10.34% (w/w), more preferably from 14.22 to 11.64% (w/w), optimally 12.93% (w/w) of citicoline.

In the composition according to the present invention, citicoline may be present as a free base or as a salt, for example in the form of sodium salt.

Preferably, the composition according to the present invention comprises from 20.27 to 13.51% (w/w), more preferably from 18.57 to 15.20% (w/w), optimally about 16.89% (w/w), of forskolin.

Forskolin is a diterpene derivative also known as coleonol; it is a substance present in the Coleus Forskohlii plant, otherwise known as Plectranthus barbatus, coleus, Indian coleus, Indian mint or false boldo.

In one embodiment of the invention, for the preparation of the composition according to the present invention, forskolin is advantageously used in the form of a dry extract of the Coleus Forskohlii root titrated at 10%.

Preferably, the composition according to the present invention comprises from 5.28 to 3.35% (w/w), more preferably from 4.48 to 3.96% (w/w), optimally about 4.40% (w/w) of homotaurine.

Preferably, the composition according to the present invention comprises from 1.21 to 0.81% (w/w), more preferably from 1.11 to 0.91% (w/w), optimally 1.01% (w/w) of riboflavin; from 8 to 5.33% (w/w), more preferably from 7.33 to 6.0% (w/w), optimally about 6.67% (w/w) of epigallocatechin gallate; from 15.51 to 10.34% (w/w), more preferably from 14.22 to 11.64% (w/w), optimally 12.93% (w/w) of citicoline; from 20.27 to 13.51% (w/w), more preferably from 18.57 to 15.20% (w/w), optimally about 16.89% (w/w) of forskolin; and from 5.28 to 3.35 % (w/w), more preferably from 4.48 to 3.96% (w/w), optimally about 4.40% (w/w) of homotaurine.

Preferably, the nutraceutical composition according to the present invention comprises at least one further component selected from the group consisting of vitamin B1, vitamin B3, vitamin B6, folic acid (B9), vitamin E, an excipient.

Preferably, in the nutraceutical composition according to the present invention said vitamin B1 is present in amounts from 0.26 to 0.18% (w/w), more preferably from 0.24 to 0.198% (w/w), optimally of about 0.198% (w/w).

Preferably, in the nutraceutical composition according to the present invention, said vitamin B3 is present in amounts from 1.42 to 0.94% (w/w), more preferably from 4.59 to 3.76% (w/w), optimally of about 4.18% (w/w).

Preferably, in the nutraceutical composition according to the present invention said vitamin B6 is present in amounts from 0.17 to 0.11% (w/w), more preferably from 0.15 to 0.13% (w/w), optimally of about 0.14% (w/w).

Preferably, in the nutraceutical composition according to the present invention said folic acid (vitamin B9) is present in amounts from 0.024 to 0.048% (w/w), more preferably from 0.033 to 0.027% (w/w), optimally of about 0.03% (w/w).

Preferably, in the nutraceutical composition according to the present invention said vitamin E is present in amounts from 5.06 to 3.37% (w/w), more preferably from 4.64 to 3.79% (w/w), optimally of about 4.22% (w/w).

Preferably, said excipient is selected from the group consisting of a lecithin, for example sunflower lecithin, a glyceride, for example glyceryl monostearate.

In a preferred embodiment, the composition is oil-based, more preferably in the form of a suspension in a vegetable oil, even more preferably in sunflower seed oil.

The composition of the invention is prepared using techniques and methods known to those skilled in the art.

According to what has been described above, by way of example, the following nutraceutical composition according to the present invention is shown:

| Component | Amount (mg/100 mg of composition) |
|---|---|
| Epigallocatechin gallate | 6.67 |
| Citicoline | 12.93 |
| Homotaurine | 4.40 |
| Forskolin | 16.89 |
| Riboflavin | 1.01 |
| Vitamin B1 | 0.22 |
| Vitamin B3 | 1.18 |
| Vitamin B6 | 0.14 |
| Folic acid | 0.03 |
| Vitamin E | 4.22 |
| Sunflower seed oil | 49.08 |
| Sunflower lecithin | 1.88 |
| Glyceryl monostearate | 1.33 |

The composition according to the present invention proved to be capable of exerting a surprisingly high neuroprotective effect, thus offering a new and effective therapeutic treatment of glaucoma.

In a further aspect, therefore, the present invention relates to the composition according to the first aspect of the invention, for use in the treatment or prevention of glaucoma.

The advantages and characteristics of this further aspect have already been highlighted with reference to the first aspect of the invention and are not repeated here.

Advantageously, the composition according to the present invention may be used for the treatment of primary open-angle glaucoma (POAG) and primary closed-angle glaucoma (PCAG).

Advantageously, the composition according to the present invention may be used for the treatment of glaucoma both alone and in association with one or more active ingredients to lower intraocular pressure, and for the prevention treatment in patients at risk of developing glaucoma.

Advantageously, the composition according to the present invention may also be used as an adjuvant to pharmacological therapy or for preventive purposes in subjects potentially capable of developing glaucoma.

The composition of the invention can be administered by systemic administration, preferably it can be administered by oral administration.

The composition of the invention may be prepared and packaged in bulk form or in the form of unit dosages.

When administered orally, the composition according to the present invention is preferably in the form of a tablet, capsule, oval tablet, pill, lozenge, powder, syrup, elixir, suspension, solution, emulsion, sachet, or cachet; more preferably the composition is in the form of a soft capsule.

According to what described above, by way of example, the following nutraceutical composition according to the present invention in the form of a soft capsule is shown:

| Component | Amount (mg/capsule) |
|---|---|
| Epigallocatechin gallate | 78.95 |
| Citicoline | 153.06 |
| Homotaurine | 52.08 |
| Forskolin | 200.00 |
| Vitamin B1 | 2.59 |
| Riboflavin | 12.00 |
| Vitamin B3 | 14.00 |
| Vitamin B6 | 1.70 |
| Folic acid | 0.40 |
| Vitamin E | 50.00 |
| Sunflower seed oil | 581.00 |
| Sunflower lecithin | 22.31 |
| Glyceryl monostearate | 15.80 |

In a further aspect, the present invention also refers to the use of riboflavin in the treatment of glaucoma, as an enhancing agent of the protective effect on retinal ganglion cells of a compound selected from the group consisting of epigallocatechin gallate, citicoline, forskolin, homotaurine, and binary, ternary, and quaternary mixtures thereof.

The advantages and characteristics of this further aspect have already been highlighted with reference to the first aspect of the invention and are not repeated here.

The invention is now illustrated through some Examples which are intended for illustrative and non-limiting purposes.

### EXPERIMENTAL PART

### Example 1 - Cell viability assay

In order to show its cytocompatibility profile, the composition according to the present invention was tested in a cell viability assay on SH-SY5Y cells, which are "neuronal-like cells", evaluating their viability after 24 hours (h) of treatment.

The choice to use SH-SY5Y cells, although they are not retinal ganglion cells, is justified by the lack of an appropriate *in vitro* model of glaucoma, and by the fact that in the literature such cells are used in *in vitro* studies of neuroprotection and oxidative stress involving the retinal neuronal component (Vernazza S, Tirendi S, Passalacqua M, Piacente F, Scarf! S, Oddone F, Bassi AM. An Innovative In Vitro Open-Angle Glaucoma Model (IVOM) Shows Changes Induced by Increased Ocular Pressure and Oxidative Stress. Int J Mol Sci. 2021 Nov 9;22(22):12129; Marín-Prida J, Pentón-Rol G, Rodrigues FP, Alberici LC, Stringhetta K, Leopoldino AM, Naal Z, Polizello AC, Llópiz-Arzuaga A, Rosa MN, Liberate JL, Santos WF, Uyemura SA, Pentón-Arias E, Curti C, Pardo-Andreu GL. C-Phycocyanin protects SH-SY5Y cells from oxidative injury, rat retina from transient ischemia and rat brain mitochondria from Ca2+/phosphate-induced impairment. Brain Res Bull. 2012 Dec 1;89(5-6):159-67; Xiong S, Xu Y, Ma M, Wang H, Wei F, Gu Q, XuX. Neuroprotective effects of a novel peptide, FK18, under oxygen-glucose deprivation in SH-SY5Y cells and retinal ischemia in rats via the Akt pathway. Neurochem Int. 2017 Sep;108:78-90). This choice is also justified by the fact that SH-SY5Y cells, as well as retinal ganglion cells, are both neuronal cells and therefore are representative of RGC cells. Furthermore, over time, doubts have emerged in the scientific field regarding the reliability and real origin of the continuous RGC-5 cell line (rat ganglion cell line) available on the market, which in the past was widely used for *in vitro* studies (Al-Ubaidi MR. RGC-5: are they really 661W? The saga continues. Exp Eye Res. 2014 Feb;119:115; Van Bergen NJ, Wood JP, Chidlow G, Trounce IA, Casson RJ, Ju WK, Weinreb RN, Crowston JG. Recharacterization of the RGC-5 retinal ganglion cell line. Invest Ophthalmol Vis Sci. 2009 Sep;50(9):4267-72).

To this end, SH-SY5Y cells were exposed for 24 hours to the preferred composition according to the present invention (INV) containing the individual ingredients according to the concentrations shown in Table 1:

**Table 1**

| Component | Amount (mg/100 mg of composition)) | Amount Tested (µg/ml) |
|---|---|---|
| Epigallocatechin gallate | 6.67 | 25.7 |
| Citicoline | 12.93 | 49.83 |
| Homotaurine | 4.40 | 16.96 |
| Forskolin | 16.89 | 65.11 |
| Riboflavin | 1.01 | 3.91 |
| Vitamin B1 | 0.22 | 0.84 |
| Vitamin B3 | 1.18 | 4.56 |
| Vitamin B6 | 0.14 | 0.55 |
| Folic acid | 0.03 | 0.13 |
| Vitamin E | 4.22 | 16.27 |

As a positive control (CTRL+), 0.01% benzalkonium chloride was used, while the negative control (CTRL-) was represented by untreated cells.

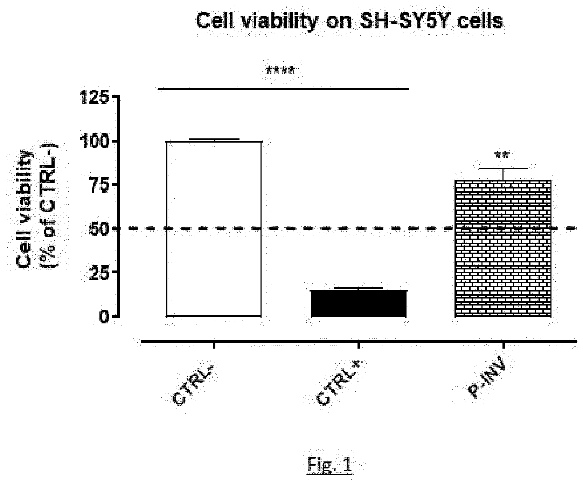

The cytotoxic activity was evaluated using 3-(4,5-dimethylthiazol-2-yl)-2,5-diphenyltetrazolium bromide (MTT), according to the ECVAM DB-ALM standard protocol no. 17. Cell viability was calculated as a percentage of CTRL-. If the viability was below 50%, the compound was identified as cytotoxic. The results are shown in Figure 1. Data represent the mean ± SEM of n=2 experiments. The statistical analysis was performed using the so-called "one sample t test" with respect to the value of 50%. **p≤0.01 vs. cut-off 50%.

From the results obtained, it was possible to conclude that the composition according to the present invention (INV) is devoid of any significant cytotoxic effects on SH-SY5Y cells vs. CTRL-.

### Example 2 - Evaluation of neuroprotection in terms of cell viability

The neuroprotective effect of the composition according to the invention (INV) was studied on the SH-SY5Y cell line. To this end, *in vitro* neurotoxicity was induced by staurosporine (STS, 250 nM) and the neuroprotective effect, in terms of cell viability, was assessed by MTT assay using a model previously described by Barrachina *et al.* (M. Barrachina, J. Secades, R. Lozano, C. Gómez-Santos, A. Santiago, I. Ferrer. Citicoline increases glutathione redox ratio and reduces caspase-3 activation and cell death in staurosporine-treated SH-SY5Y human neuroblastoma cells. Brain Res., 2002, 957(1):84-90), with some modifications, such as the concentration and incubation time with staurosporine, as detailed in the following protocol:
- from t=0 to t=6 hours: cells pretreatment with the compositions to be tested;
- from t=6 to t=24 hours: induction of neurotoxicity on cells with STS (250 nM) and co-treatment with the compositions to be tested;
- t=24 hours: MTT assay, as defined above.

For the test, the following three compositions were used:
- INV: composition according to the invention, as shown in Table 1 of Example1
- B2: riboflavin (3.91 µg/ml. Dilution tested *in vitro* and corresponding to a concentration of 12 mg/capsule);
- CONF: composition according to the invention, as shown in Table 1 of Example 1, but without riboflavin;

in order to highlight, through the comparison of the results obtained with the INV composition to the sum of the results obtained by B2 + CONF, the synergistic effect deriving from the combination of riboflavin with the other compounds already known for the treatment of glaucoma (epigallocatechin gallate, citicoline, forskolin, and homotaurine).

Cells treated only with STS were used as a control (CTRL).

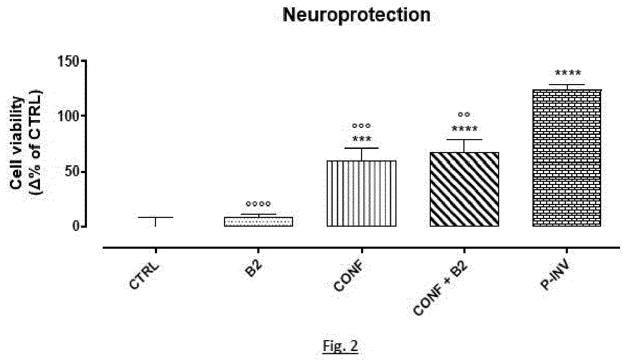

The results are shown in Figure 2, where the data represent the mean ± SEM of n=3 experiments and are expressed as a percentage of the CTRL which is assumed as zero. Statistical analysis was performed using One-way ANOVA test, followed by Dunnett's post hoc test. ****p≤0.0001, ***p≤0.001 vs. CTRL; °°°°p≤0.0001, °°°p≤0.001, °°p≤0.01 vs. INV.

The results showed that the neuroprotective activity exerted by the composition according to the invention (% cell viability by MTT assay = 124.2), was far greater than the sum of the neuroprotective activity exerted by riboflavin alone (% cell viability by MTT assay = 8.49) and by a composition according to the invention but without riboflavin (% vitality by MTT assay = 58.8).

This confirmed the existence of a synergistic effect deriving from the combined use of riboflavin with other compounds already known for the treatment of glaucoma (epigallocatechin gallate, citicoline, forskolin, and homotaurine).

### Example 3 - Evaluation of neuroprotection in terms of BDNF expression

To further evaluate the neuroprotective effect of the composition according to the invention, another test was performed on SH-SY5Y cells by enzyme-linked immunosorbent assay (ELISA), to evaluate the expression of BDNF, a known neuroprotective factor (E.M Gonulalan, O. Bayazeid , F. Yalcin, L.O Demirezer. The roles of valerenic acid on BDNF expression in the SH-SY5Y cell. Saudi Pharm J., 2018, 26(7):960-964; W.J. Lee, G.H. Lee, J. Hur, H.G. Lee, E. Kim, et al., Taurine and Ginsenoside Rf Induce BDNF Expression in SH-SY5Y Cells: A Potential Role of BDNF in Corticosterone-Triggered Cellular Damage. Molecules, 2020;25(12):2819).

To this end, *in vitro* neurotoxicity was induced by STS (100 nM) and BDNF expression was measured by ELISA assay.

The protocol used for the test was the following:
- from t=0 to t=6 hours: cells pretreatment with the composition according to the invention (INV);
- from t=6 to t=24 hours: induction of neurotoxicity on cells with STS (100 nM) and co-treatment with the composition according to the invention (INV);
- t=24 hours: lysis of the samples with RIPA buffer and subsequent ELISA test on these lysates to evaluate the BDNF expression. Furthermore, the total protein concentration was determined for each lysate by BCA assay, according to the protocol provided by the manufacturer.

For the test, the composition according to the invention from Table 1 of Example 1 (INV) was used. Cells treated only with STS were used as a control (CTRL).

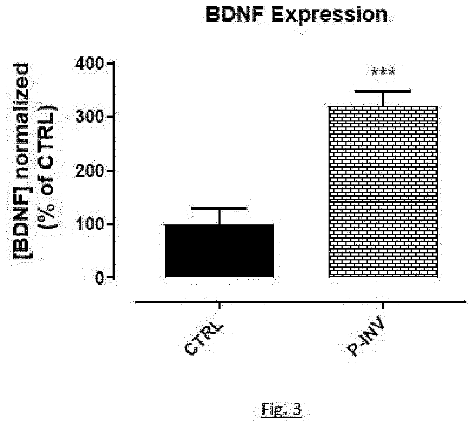

The results obtained are shown in Figure 3. Data are represented as mean ± SEM of n=3 experiments, normalized by the total protein amount from each lysed sample and compared to the CTRL. Statistical analysis was performed using the unpaired samples t-test. ***p≤0.001.

The results showed that the composition according to the present invention was able to increase the expression of BDNF when compared to CTRL thus demonstrating a neuroprotective effect.

### Example 4 - Evaluation of riboflavin neuroprotective effect in the composition of the invention

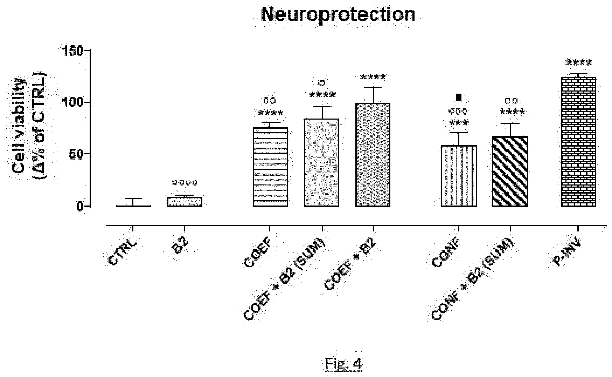

The aim of the present study was to investigate neuroprotection in an *in vitro* model already described in the literature (Barrachina, J. Secades, R. Lozano, C. Gómez-Santos, S. Ambrosio, and I. Ferrer, Citicoline increases glutathione redox ratio and reduces caspase-3 activation and cell death in staurosporine-treated SH-SY5Y human neuroblastoma cells, Brain Res 957, 84, 2002; D. Jantas, M. Pytel, J. W. Mozrzymas, M. Leskiewicz, M. Regulska, L. Antkiewicz-Michaluk, and W. Lason, The attenuating effect of memantine on staurosporine-, salsolinol- and doxorubicin-induced apoptosis in human neuroblastoma SH-SY5Y cells, Neurochem Int 52, 864, 2008), if the addition of riboflavin (vitamin B2) to a mixture of citicoline, homotaurine, epigallocatechin gallate and forskolin (*i.e*. COEF), or to a mixture of citicoline base, homotaurine, epigallocatechin gallate, forskolin and vitamins B1, B3, B6, B9 and E (*i.e*. CONF), could have an additive or synergistic effect compared to mixtures without vitamin B2 (*i.e*. COEF and CONF) (Figure 4).

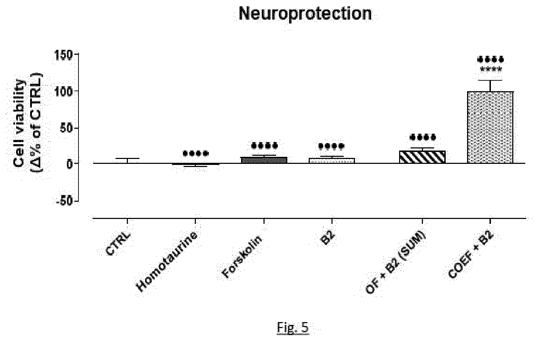

The present study also aimed to evaluate the effect of B2 when added to homotaurine and forskolin (Figure 5).

To this end, the same assay system reported in Example 2 was used.

In detail, SH-SY5Y cells were pretreated for 6 hours with single test elements or with the following mixtures:
- B2 (riboflavin): 3.91 µg/ml;
- Homotaurine: 16.96 µg/ml;
- Forskolin: 65.11 µg/ml.
- COEF: Citicoline, homotaurine, EGCG, and forskolin
- COEF+B2: Citicoline, homotaurine, EGCG, forskolin, and vitamin B2 (B2) *i.e*. the composition of the invention
- CONF: Citicoline, homotaurine, EGCG, forskolin, and vitamins B1, B3, B6, B9 and E.
- P-INV: Citicoline, homotaurine, EGCG, forskolin, and vitamins B1, B2, B3, B6, B9 and E, *i.e*. the preferred composition of the invention.

In detail, as shown in Figure 4, P-INV showed the best performance in terms of neuroprotection as regard to B2 (°°°° p≤0.0001), COEF (°°p≤0.01) and CONF (°°°p≤0.001), thus demonstrating the synergistic effect of B2 when added to CONF, as emerged from the comparison with CONF + B2 (SUM) (°°p≤0.01). Furthermore, the addition of B2 to the COEF (*i.e*. COEF + B2) endowed the resulting mixture with a greater neuroprotective effect when compared to COEF and to the arithmetic sum of COEF and B2 [*i.e*. COEF + B2 (SUM)] even if in a non-statistically significant manner (Figure 4).

Finally, COEF + B2 showed a greater neuroprotective effect than CONF (▪p≤0.05, Figure 4).

As shown in Figure 5, the same study highlighted that none of the individual ingredients tested (*i.e.*, homotaurine, forskolin and B2) were significantly different from CTRL unlike COEF + B2 which showed a neuroprotective effect *vs*. CTRL (****p≤0.0001).

Furthermore, COEF + B2 showed a statistically greater neuroprotective effect than the individual ingredients or the arithmetic sum thereof [*i.e*. OF + B2 (SUM)] (••••p≤0.0001, Figure 5).

Overall, given the results of the experiments performed to determine the activity of the mixtures COEF (*i.e*., citicoline, homotaurine, EGCG and forskolin), COEF + B2 (*i.e*., citicoline, homotaurine, EGCG, forskolin and vitamin B2), CONF (*i.e*., citicoline, homotaurine, EGCG, forskolin, and vitamins B1, B3, B6, B9 and E) and P-INV (*i.e*. citicoline, homotaurine, EGCG, forskolin, and vitamins B1, B2, B3, B6, B9 and E), it was apparent that the addition of vitamin B2, at the concentration shown in Table 1, to the COEF and CONF mixtures synergistically enhanced the protective effect (Figure 4).

## Claims

1. A nutraceutical composition comprising riboflavin, epigallocatechin gallate, citicoline, forskolin, and homotaurine.

2. The nutraceutical composition according to claim 1, comprising from 1.21 to 0.81% (w/w), more preferably from 1.11 to 0.91% (w/w), optimally 1.01% (w/w) of riboflavin.

3. The nutraceutical composition according to claim 1 or 2, comprising from 8 to 5.33% (w/w), more preferably from 7.33 to 6.0% (w/w), optimally about 6.67% (w/w), of epigallocatechin gallate.

4. The nutraceutical composition according to any one of claims 1 to 3, comprising from 15.51 to 10.34% (w/w), more preferably from 14.22 to 11.64% (w/w), optimally 12.93% (w/w) of citicoline.

5. The nutraceutical composition according to any one of claims 1 to 4, comprising from 20.27 to 13.51% (w/w), more preferably from 18.57 to 15.20% (w/w) optimally about 16.89% (w/w) of forskolin.

6. The nutraceutical composition according to any one of claims from 1 to 5, comprising from 5.28 to 3.35% (w/w), more preferably from 4.48 to 3.96% (w/w), optimally about 4.40% (w/w) of homotaurine.

7. The nutraceutical composition according to any one of claims 1 to 6, wherein said composition is oil-based.

8. The nutraceutical composition according to any one of claims 1 to 7, wherein said composition is in the form of a suspension.

9. The nutraceutical composition according to any one of claims 1 to 8, consisting of:
| Component | Amount (mg/100 mg of composition) |
|---|---|
| Epigallocatechin gallate | 6.67 |
| Citicoline | 12.93 |
| Homotaurine | 4.40 |
| Forskolin | 16.89 |
| Riboflavin | 1.01 |
| Vitamin B1 | 0.22 |
| Vitamin B3 | 1.18 |
| Vitamin B6 | 0.14 |
| Folic acid | 0.03 |
| Vitamin E | 4.22 |
| Sunflower seed oil | 49.08 |
| Sunflower lecithin | 1.88 |
| Glyceryl monostearate | 1.33 |

10. A composition according to any one of claims 1 to 9, for use in the treatment or in the prevention of glaucoma.

11. The composition for use according to claim 10, for use in the treatment of primary open-angle glaucoma (POAG) or primary closed-angle glaucoma (PCAG).

12. The composition for use according to claim 10 or 11, for use in the treatment of glaucoma alone, in association with one or more active ingredients to lower intraocular pressure, or for use in the prevention in patients at risk of developing glaucoma.

13. The composition for use according to any one of claims 10 to 12, as an adjuvant to drug therapy or for preventive purposes in subjects potentially capable of developing glaucoma.

14. The composition for use according to any one of claims 10 to 13, wherein said composition is administered orally.

15. The composition for use according to any one of claims 10 to 14, in bulk form or in unit dosage form.

16. The composition for use according to any one of claims 10 to 15, in the form of a tablet, capsule, oval tablet, pill, lozenge, powder, syrup, elixir, suspension, solution, emulsion, sachet, or cachet.

17. The composition for use according to any one of claims 10 to 16, in the form of a soft capsule.
